# EUROPÄISCHE PATENTANMELDUNG

(11) **EP 1 080 715 A2**
(43) Veröffentlichungstag der Anmeldung: **07.03.2001**
(21) Anmeldenummer: 00115538.1
(22) Anmeldetag: 19.07.2000
(51) Int. Cl.: A61K 7/32, A61K 7/48

(54) **Wirkstoffkombination und Fusspflegezubereitungen dieselben enthaltend**

(30) Priorität: 05.08.1999 DE 19936968
(71) Anmelder: Bode Chemie GmbH & Co., D-22525 Hamburg (DE)
(72) Erfinder: Pietsch, Hanns, Dr., 20148 Hamburg (DE); Knieler, Roland, Dr., 22529 Hamburg (DE); Rudolf, Marco, Dr., 22299 Hamburg (DE); Holling, Jaana, 20251 Hamburg (DE)
(74) Vertreter: Dinné, Erlend

(57) **Zusammenfassung**

Synergistische Wirkstoffkombinationen aus
a) mindestens einer quaternären Ammoniumverbindung gewählt aus der Gruppe, welche gebildet wird aus quaternären Ammoniumverbindungen der allgemeinen Strukturformel 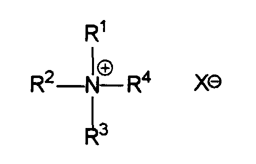 und Alkylpyridiniumsalzen der allgemeinen Strukturformel 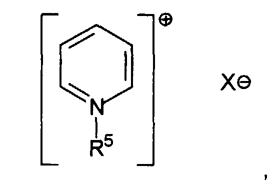 und
b) mindestens einem anorganischen Antitranspirantwirkstoff,
   wobei R¹ und R² unabhängig voneinander Methyl- oder Ethylgruppen darstellen, R³ gewählt wird aus der Gruppe der Alkylreste mit 1 bis 18 Kohlenstoffatomen, R⁴ gewählt wird aus der Gruppe, welche gebildet wird aus den Alkylresten mit 8 bis 18 Kohlenstoffatomen und den Aralkylresten mit 7 bis 18 Kohlenstoffatomen, R⁵ wird gewählt aus der Gruppe der Alkylreste mit 10 bis 18 Kohlenstoffatomen und worin X⁻ ein anorganisches oder organisches Anion darstellt.

## Beschreibung

Die vorliegende Erfindung betrifft Wiirkstoffkombinationen von an sich bekannten Substanzen. Insbesondere betrifft die Erfindung Fußpflegezubereitungen mit antitranspiranten, bakteriziden und fungiziden Eigenschaften, die diese Wirkstoffkombinationen enthalten.

Körpergerüche in ihrem ursprünglichen Sinn dienen der sozialen Kommunikation. Beispielsweise haben spezielle, von den Duftdrüsen abgesonderte Stoffe (Pheromone) in der Tierwelt einen wesentlichen Einfluß auf Sexual-, Territorial- und Aggressionsverhalten oder sind Ausdruck von Individualität bzw. Rangordnung. Für den Menschen hat diese Art von Verständigung jedoch sehr stark an Bedeutung verloren, wie die vergleichsweise kümmerliche Leistungsfähigkeit des menschlichen Geruchssinns andeutet. Körpergeruch wird in modernen, westlich geprägten Industriegesellschaften als aufdringlich, abstoßend und ungepflegt angesehen. Die Anwendung von kosmetischen oder dermatologischen Desodorantien bietet eine Möglichkeit, unangenehmem Körpergeruch entgegenzuwirken.

Körpergeruch entsteht, wenn der an sich geruchlose frische Schweiß durch Mikroorganismen zersetzt wird. Die grampositiven Bakterien bauen das Sebum, Eiweißstoffe und die gelösten Bestandteile des Schweißes ab. Dabei werden geruchsintensive kurzkettige Fettsäuren und Amine frei. Insbesondere der apokrine Schweiß transportiert auf seinem Weg über die Talgdrüsen und Haarfollikel eine Reihe von Mikroorganismen an die Hautoberfläche, die recht bald in die Zersetzung eingreifen. Auf der Haut befindet sich aber auch eine bestimmte residente Mikroorganismenpopulation, welche, ergänzt durch transiente Mikroorganismen aus der Umwelt den Abbau des Schweißes beeinflußt.

Den üblichen kosmetischen Desodorantien liegen unterschiedliche Wirkprinzipien zugrunde.

Übliche in Desodorantien eingesetzte Wirkstoffe besitzen beispielsweise antimikrobielle Eigenschaften, die die mikrobielle Zersetzung des frischen Schweißes verhindern. Eine desodorierende Wirkung läßt sich aber auch durch enzyminhibierende, aber nicht antimikrobiell wirkende Substanzen und/oder durch Ionenaustauscher erreichen. Kationenaustauscher z. B. sollen Ammoniak, Indol und ähnliche Gerüche absorbieren. Wirkstoffe mit antitranspiranten Eigenschaften hingegen sind solche, die das Schwitzen des menschlichen Körpers reduzieren oder ganz unterdrücken, wobei die Wirkung bei topischer Applikation in der Regel lokal ist.

Die in sogenannten Antitranspirantien eingesetzten Wirkstoffe hemmen in erster Linie die Schweißsekretion, haben aber im allgemeinen auch eine gewisse antibakterielle Wirkung. Sie wirken blockierend auf den Teil des Ausführungsganges der Schweißdrüsen, der durch das Stratum corneum führt. Sie verengen bzw. verschließen die Schweißdrüsen, so daß das Schwitzen ganz oder teilweise unterdrückt wird.

Als antitranspirante Wirkstoffe sind Aluminiumsalze seit langem bekannt. Aluminiumchlorid, Aluminiumchlorhydrat, -nitrat, -sulfat, -acetat usw. werden seit einigen Jahren als antitranspirante oder antiperspirante Wirkstoffe verwendet. Daneben finden auch Zink-, Magnesium- und Zirkoniumverbindungen Anwendung. Der Stand der Technik wird beispielsweise beschrieben in: *H. P. Fiedler, Der Schweiß, Editio Cantor, Aulendorf, 2. Auflage, S. 303-377, Kapitel K:* 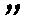 *Mittel zur Hemmung der Transpiration"*. Von den dort als Antitranspirant-Wirkstoffe beschriebenen Metallverbindungen haben sich für die Anwendung in kosmetischen und dermatologischen Antitranspirantien nur die Aluminiumsalze und ― in etwas geringerem Maße ― die Aluminium/Zirkoniumsalze durchgesetzt.

Der Hauptnachteil der sehr wirksamen Aluminiumchloridlösungen ist, daß sie stark sauer reagieren und daher mitunter ein Brennen auf der Haut und eine Rötung verursachen können, welche eventuell mit Abschilferung verbunden sein kann. Aus diesem Grund setzt man üblicherweise die teilneutralisierten, besser hautverträglichen, aber nicht ganz so wirksamen Aluminiumhydroxychloride ein, die auch einen etwas günstigeren pH-Wert von etwa 4 aufweisen. Allerdings können auch diese bei häufiger Anwendung und bei empfindlichen Personen Hautschäden hervorrufen oder zu unerwünschten Verfärbungen auf Textilien führen. Letzteres kann beispielsweise durch die Verwendung von Zirkonium/Aluminium-Mischsalzen verringert werden.

Die Haut der Füße bedarf wegen ihrer besonderen Beanspruchung spezieller Pflegemittel. An den Füße befinden sich ekkrine Schweißdrüsen, die tätig werden, sobald die Hauttemperatur 31 °C übersteigt. Durch enges Schuhwerk, schlecht durchlässiges und nicht aufsaugendes Schuhmaterial (z. B. Kunstleder oder Gummi) oder Strümpfe aus synthetischem Material wird ein Temperaturanstieg begünstigt. Da der abgesonderte Schweiß nicht verdunsten kann, entsteht eine 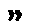 Treibhausatmosphäre". In diesem feuchtwarmen Milieu sind die schweißzersetzenden Bakterien besonders aktiv, so daß ein intensiver unangenehmer Geruch entstehen kann, welcher von Schuhen und Strümpfen angenommen wird.

Dem Fußschweiß ― und den damit verbundenen Geruchsproblemen ― kann durch die Verwendung von Fußdesodorantien oder Antitranspirantien mehr oder weniger wirksam begegnet werden.

Fußpflegepräparate sollten im Idealfall das Entstehen von Fußschweiß ganz oder teilweise verhindern und die Zersetzung von Schweiß und anderen Hautbestandteilen durch Bakterien und Hautpilze und eine Besiedelung der Füße durch diese Mikroorganismen unterdrücken oder gänzlich verhindern. Ferner wünschenswert sind insbesondere auch pflegende Bestandteile wie rückfettende und/oder feuchtigkeitspendende Inhaltsstoffe.

Neben den rein kosmetischen Fußpflegemitteln finden auch dermatologische Präparate Verwendung, welche Fußbeschwerden lindern und möglicherweise sogar der Entstehung von Fußerkrankungen vorbeugen können. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z. B. Kosmetikverordnung, Lebensmittel- und Arzneimittelgesetz).

Dermatomycosen sind Krankheiten, bei der gewisse Pilzarten, insbesondere Dermatophyten, in die Haut und Haarfollikel eindringen. Die Symptome von Dermatomycosen sind beispielsweise Bläschen, Exfoliation, Rhagaden und Erosion, meist verbunden mit Juckreiz oder allergischem Ekzem.

Alle Bereiche der menschlichen Haut können von Dermatomycosen befallen werden. Besonders häufig sind allerdings die Körperbereiche betroffen, auf welchen sich durch Kleidung, Schmuck oder Schuhwerk Feuchtigkeit und Wärme stauen können. So gehört der Fußpilz zu den bekanntesten und am weitesten verbreiteten Dermatomycosen. Mit Fußpilz kann man sich durch Barfußlaufen in Duschräumen und Sport- und Badeanlagen sehr leicht infizieren. Er verursacht einen lästigen Juckreiz, insbesondere zwischen den Zehen, und sogar schmerzhafte Ekzeme, die sich über den ganzen Fuß erstrecken können. Auch nach scheinbarer Abheilung können die Pilzsporen noch nach 18 Monaten eine Zweitinfektion hervorrufen.

Besonders unangenehm sind weiterhin Pilzerkrankungen der Finger- und Fußnägelbereiche (Onychomycosen).

Es kann daher durchaus wünschenswert sein, wenn kosmetische oder dermatologische Fußpflegezubereitungen auch eine fungistatische bzw. fungizide Wirksamkeit haben, um beispielsweise einer Fußpilzerkrankung vorzubeugen bzw. eine Zweitinfektion zu verhindern.

Für die Fußpflege sind eine Reihe von antitranspiranten Zubereitungen an sich bekannt. Diese enthalten als antitranspiranten Wirkstoff in der Regel Aluminiumchlorid, Aluminiumchlorhydrat und/oder Zirkoniumchlorid.

Antitranspirantien, welche Aluminiumhydroxychloride als Wirkstoffe enthalten, werden vor allem in Form von wäßrigen Lösungen und als Pulver oder Granulate (Trockensprays) eingesetzt. So werden im Bereich der Fußpflege beispielsweise Fußpuder angewendet, die ― nach der Fußwäsche angewendet und/oder in Strümpfe und Schuhe eingestreut ― hautglättend, kühlend, feuchtigkeitsaufsaugend, schweißhemmend, antiseptisch, desodorierend und gegebenenfalls hornhauterweichend wirken sollen.

Auch andere Zubereitungsformen, wie z. B. alkoholische Lösungen, Stiftformulierungen, Emulsionen, Mikroemulsionen, Cremes, Salben, Roller und dergleichen, sind prinzipiell denkbar, allerdings teilweise schwierig stabil zu formulieren.

Für Stifte auf Basis von Natriumstearat oder anderen Seifen beispielsweise sind die sauren Aluminiumsalze nicht geeignet, da sie zum Aussalzen der Seife und somit zu chemischer Unverträglichkeit führen. Bei größeren Zusatzmengen, die für Antitranspirant-Stifte notwendig sind, wird außerdem die Bildung eines festen Seifengels verhindert.

Sollen kosmetische oder pharmazeutische Zubereitungen bestimmte Wirkstoffe enthalten, ist es prinzipiell immer denkbar, daß die übrigen Bestandteile mit den Wirkstoffen nicht kompatibel sind. Aus diesen und aus Gründen der Verträglichkeit ist es daher stets zu bevorzugen, die Einsatzkonzentrationen der Wirkstoffe möglichst niedrig zu halten, selbst bei Verwendung an sich unbedenklicher Substanzen.

Aufgabe der vorliegenden Erfindung war es, den Nachteilen des Standes der Technik abzuhelfen und Wirkstoffe bzw. Wirkstoffkombination zu finden, welche eine gute antitranspirante und mikrobizide Wirkung zeigen, nicht so stark sauer sind, eine gute Hautverträglichkeit aufweisen und Textilien nicht schädigen oder verfärben. Ferner sollen die Stoffe oder Kombinationen bakteriostatisch, besser noch bakterizid, fungistatisch bzw. fungizid wirken und sich gut in die üblichen Darreichungsformen wie Lösung, Milch, Lotion, Creme, Salbe usw. einarbeiten lassen.

Es war überraschend und für den Fachmann in keiner Weise vorhersehbar, daß synergistische Wirkstoffkombinationen aus
a) mindestens einer quaternären Ammoniumverbindung gewählt aus der Gruppe, welche gebildet wird aus quaternären Ammoniumverbindungen der allgemeinen Strukturformel 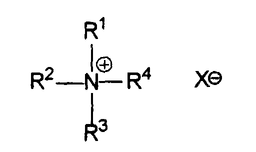 und Alkylpyridiniumsalzen der allgemeinen Strukturformel 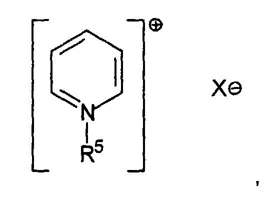 und
b) mindestens einem anorganischen Antitranspirantwirkstoff
   die Nachteile des Standes der Technik beseitigen.

Die erfindungsgemäßen Wirkstoffkombinationen wirken erstaunlicherweise in synergistischer Weise, also überadditiv in bezug auf die Einzelkomponenten.

Erfindungsgemäß ist auch die Verwendung solcher Wirkstoffkombinationen als antibakterielle (bakteriostatische bzw. bakterizide), antimycotische, fungistatische und/oder fungizide Wirkstoffe.

R¹ und R² stellen unabhängig voneinander Methyl- oder Ethylgruppen dar, R³ wird gewählt aus der Gruppe der Alkylreste mit 1 bis 18 Kohlenstoffatomen, R⁴ wird gewählt aus der Gruppe, weiche gebildet wird aus den Alkylresten mit 8 bis 18 Kohlenstoffatomen und den Aralkylresten mit 7 bis 18 Kohlenstoffatomen, und R⁵ wird gewählt aus der Gruppe der Alkylreste mit 10 bis 18 Kohlenstoffatomen.

X⁻ stellt ein anorganisches oder organisches Anion dar, beispielsweise ein Halogenid (z. B. Chlorid oder Bromid), ferner ein anorganisches Oxo-Element-Anion (von diesen insbesondere Sulfat, Carbonat, Phosphat, Borat und Aluminat) sowie ein Alkylsulfat (insbesondere Ethylsulfat) oder z. B. ein Lactat, Acetat, Benzoat, Propionat, Tartrat, Citrat und andere mehr.

Bevorzugt im Sinne der vorliegenden Erfindung sind quaternäre Ammoniumverbindungen, in denen die Reste R¹, R², R³ und R⁴ rein aliphatische Verbindungen darstellen und X⁻ kein Halogenid ist.

Quaternäre Ammoniumverbindungen sind handelsübliche Produkte und werden z. B. als bakterizide und fungizide Wirkstoffe zur Herstellung von Desinfektionsmitteln eingesetzt, sie sind oberflächenaktiv und werden als Netzmittel oder Emulgierhilfsmittel verwendet sowie im Bereich der Textilindustrie zur Avivage (= Griffigmachen) von Textilien.

Auch die Verwendung von quaternären Ammoniumverbindungen in kosmetischen oder dermatologischen Zubereitungen ist an sich bekannt. Sie werden bevorzugt zur Herstellung konditionierend wirkender Haarpflegemittel verwendet. Wesentliche Eigenschaften von Haarpflegemitteln, wie beispielsweise die Verbesserung von Kämmbarkeit und Griff sowie die Verhinderung statischer Aufladung der Haare werden durch den Einsatz von quaternären Ammoniumverbindungen verbessert.

Zwar werden in *H. P. Fiedler, Der Schweiß (Editio Cantor, Aulendorf, 2. Auflage, S. 358 ff,* 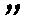 *Physiologische Antiperspirants")* auch einige spezielle quaternäre Ammoniumverbindungen als physiologisch wirksame Antitranspirantien genannt. Allerdings werden diese nicht topisch angewendet, sondern auf iontophoretischen Wege in die Haut eingeschleust (d. h. unter dem Einfluß des elektrischen Stromes durch die Haut in den Körper gebracht), und ferner haben diese Verbindungen entweder nur kurzkettige Reste R¹, R², R³ und R⁴ (Methyl-, Ethyl- und/oder Butyl-) oder zusätzlich solche, die eine OH-Gruppe tragen (wie beispielsweise das dort genannte Butyldimethyl(benzoylethanol)-ammoniumbromid). Nach der a. o. O. dargestellten Tabelle zeigen die untersuchten quaternären Ammoniumverbindungen zudem völlig unterschiedliche Wirksamkeiten, so daß der Stand der Technik keinerlei Hinweise geben konnte, welche Rückschlüsse auf die erfindungsgemäßen Wirkstoffkombinationen erlaubt hätten.

Erfindungsgemäß bevorzugt ist es, die quaternäre(n) Ammoniumverbindung(en) aus der Gruppe Benzalkoniumchlorid (N-Alkyl-N,N-dimethylbenzylammoniumchlorid), Cetrimoniumbromid (N-Hexadecyl-N,N,N-trimethyl-ammoniumbromid), Cetyltrimethylammoniumbromid, Mecetroniumethylsulfat (N-Hexadecyl-N-ethyl-N,N-dimethylammoniumethylsulfat), N,N-Didecyl-N,N-dimethylammoniumchlorid und N,N-Dioctyl-N,N-dimethylammoniumchlorid zu wählen. Ferner vorteilhaft werden die Substanzen gewählt aus der Gruppe der Benzyltrialkylammoniumchloride oder -bromide, wie beispielsweise Benzyldimethylstearylammoniumchlorid. Erfindungsgemäß bevorzugt sind ferner Lauryl- oder Cetylpyrimidiniumchlorid.

Erfindungsgemäß ganz besonders bevorzugt ist Hexadecyldimethylethylammoniumethylsulfat. Dieses zeichnet sich zudem insbesondere durch eine sehr gute Hautverträglichkeit aus.

Die quaternären Ammoniumverbindungen können in den Wirkstoffkombinationen vorteilhaft sowohl einzeln als auch im Gemisch vorliegen.

Es ist vorteilhaft, den oder die anorganischen Antitranspirantwirkstoff(e) aus der Gruppe Aluminiumchlorid, Aluminiumhydroxychlorid, Aluminium-Zirkoniumchlorid, Aluminium-Zirkonium-Chlorhydrat, Aluminium-Zirkoniumsulfat, Aluminiumnitrat, -lactat, -allantoinat, -acetat, -sulfat, Zinkchlorid, -sulfat, -nitrat, -acetat, -lactat und -allantoinat zu wählen.

Die erfindungsgemäßen Wirkstoffkombinationen eignen sich hervorragend für die Verwendung als desodorierender Wirkstoff in kosmetischen Desodorantien sowie gegen unreine Haut, leichte Formen der Akne bzw. Propionibakterium acnes.

Ferner hat sich herausgestellt, daß die erfindungsgemäßen Wirkstoffkombinationen den Verderb organischer Substanz, insbesondere kosmetischer und dermatologischer Zubereitungen, durch den Befall mit Mykobionten verhindern können, wenn sie diesen Zubereitungen zugesetzt werden.

Erfindungsgemäß sind somit auch ein Verfahren zur Bekämpfung von Mykobionten, dadurch gekennzeichnet, daß die erfindungsgemäßen Wirkstoffkombinationen gegebenenfalls in einem geeigneten kosmetischen oder dermatologischen Träger, mit dem durch Mykobionten kontaminierten Bereich in Kontakt gebracht werden, sowie ein Verfahren zum Schutze organischer Produkte vor dem Befall mit Mykobionten, dadurch gekennzeichnet, daß diesen organischen Produkten die erfindungsgemäßen Wirkstoffkombinationen in wirksamer Menge zugegeben werden.

Der Stand der Technik lieferte nicht den geringsten Hinweis auf die erfindungsgemäße Verwendung als antimycotisches Wirkprinzip.

Erfindungsgemäß werden die Wirkstoffkombinationen bevorzugt in kosmetischen oder dermatologischen Zusammensetzungen in einer Menge von 0,5 bis 30,0 Gew.-%, insbesondere von 1 bis 30,0 Gew.-%, bezogen auf das, Gesamtgewicht der Zusammensetzung, eingesetzt Vorteilhaft enthalten die Zusammensetzungen zwischen 0,01 und 2,0 Gew.-% von einer oder mehreren quaternären Ammoniumverbindung(en) und zwischen 5,0 und 30 Gew.-% eines oder mehrerer anorganischer Antitranspirantwirkstoffe, jeweils bezogen auf das Gesamtgewicht der Zusammensetzung. Liegen die kosmetischen oder dermatologischen Zusammensetzungen in Form wäßriger Lösungen ohne zusätzliche Hautpflegesubstanzen vor, so ist es vorteilhaft, wenn der Gehalt an Aluminiumchlorhydrat nicht über 20 Gew.-% liegt (bezogen auf das Gesamtgewicht der wäßrigen Lösung).

Es ist insbesondere vorteilhaft, das Verhältnis von quaternärer Ammoniumverbindung (eine oder mehrere Verbindungen) zu anorganischem Antitranspirantwirkstoff (eine oder mehrere Verbindungen) in der Wirkstoffkombination aus dem Bereich 3 : 997 bis 30 : 70 zu wählen.

Es ist auch von Vorteil, anstatt reiner erfindungsgemäßer Einzelkomponenten solche Stoffe zu verwenden, welche sich ihrerseits durch einen Gehalt an erfindungsgemäßen Wirkstoffkomponenten auszeichnen.

Die erfindungsgemäßen Wirkstoffkombinationen lassen sich ohne Schwierigkeiten in gängige kosmetische oder dermatologische Formulierungen einarbeiten, vorteilhaft in Pumpsprays, Aerosolsprays, Crèmes, Salben, Tinkturen, Lotionen, Nagelpflegeprodukte (z. B. Nagelbalsame und Produkte, die zur Heilung von Nagelmykosen geeignet sind) und dergleichen.

Ganz besonders vorteilhaft liegen die erfindungsgemäßen Wirkstoffkombinationen in Form von Fußpflegemittel vor.

Es ist auch möglich und gegebenenfalls vorteilhaft, die erfindungsgemäßen Wirkstoffkombinationen mit anderen Wirkstoffen zu kombinieren, beispielsweise mit anderen antimikrobiell, antimycotisch bzw. antiviral wirksamen Stoffen.

Besonders vorteilhaft sind einfache alkoholische und wäßrige Lösungen, beispielsweise in Ethanol, Propanol-1 sowie Propanol-2, insbesondere solche, welche eine Wirkstoffkonzentration von mehr 0,1 Gew.-%, bezogen auf das Gesamtgewicht der Zusammensetzung, aufweisen. Ebenfalls vorteilhaft ist die erfindungsgemäße Verwendung der Wirkstoffe in Emulsionen und Cremes, welche mindestens eine Fettphase enthalten.

Stellen die erfindungsgemäß erhaltenen Zubereitungen Desodorantien/Antitranspirantien dar, so können zusätzlich zu den erfindungsgemäßen Wirkstoffkombinationen alle gängigen Wirkstoffe vorteilhaft genutzt werden, beispielsweise Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit, Kaolinit, llit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäß erhaltenen Zubereitungen eingearbeitet zu werden. Vorteilhafte Substanzen sind zum Beispiel 2,4,4'-Trichlor-2'-hydroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatriën-1-ol) sowie die in den Patentoffenlegungsschriften DE-37 40 186, DE-39 38 140, DE-42 04 321, DE-42 29 707, DE-42 29 737, DE-42 37 081, DE-43 09 372, DE-43 24 219 beschriebenen wirksamen Agenzien.

Entsprechend der erfindungsgemäßen Verwendung können die kosmetischen Zubereitungen als Desodorantien bzw. Antitranspirantien bezeichnet werden. Vorteilhaft können sie beispielsweise in Form von Aerosolen, also aus Aerosolbehältern, Quetschflaschen oder durch eine Pumpvorrichtung versprühbaren Präparaten vorliegen oder in Form von mittels Roll-on-Vorrichtungen auftragbaren flüssigen Zusammensetzungen, als Deo-Stifte (Deo-Sticks) und in Form von aus normalen Flaschen und Behältern auftragbaren W/O- oder O/W-Emulsionen, z. B. Crèmes oder Lotionen. Weiterhin können die kosmetischen Desodorantien vorteilhaft in Form von desodorierenden bzw. antitranspirierend wirkenden Tinkturen, desodorierenden bzw. antitranspirierend wirkenden Intimreinigungsmitteln, desodorierenden bzw. antitranspirierend wirkenden Pudern oder deodorierenden bzw. antitranspirierend wirkenden Pudersprays vorliegen.

Als Treibmittel für aus Aerosolbehältern versprühbare kosmetische und/oder dermatologische Zubereitungen im Sinne der vorliegenden Erfindung sind die üblichen bekannten leichtflüchtigen, verflüssigten Treibmittel, beispielsweise Kohlenwasserstoffe (Propan, Butan, Isobutan) geeignet, die allein oder in Mischung miteinander eingesetzt werden können. Auch Druckluft ist vorteilhaft zu verwenden.

Natürlich weiß der Fachmann, daß es an sich nichttoxische Treibgase gibt, die grundsätzlich für die Verwirklichung der vorliegenden Erfindung in Form von Aerosolpräparaten geeignet wären, auf die aber dennoch wegen bedenklicher Wirkung auf die Umwelt oder sonstiger Begleitumstände verzichtet werden sollte, insbesondere Fluorkohlenwasserstoffe und Fluorchlorkohlenwasserstoffe (FCKW).

Als übliche kosmetische Trägerstoffe zur Herstellung von desodorierenden bzw. antitranspirierend wirkenden Zubereitungen mit einem Gehalt an erfindungsgemäßen Wirkstoffkombinationen können neben Wasser, Ethanol und Isopropanol, Glycerin und Propylenglykol hautpflegende Fett- oder fettähnliche Stoffe, wie Ölsäuredecylester, Cetylalkohol, Cetylstearylalkohol und 2-Octyldodecanol, in den für solche Präparate üblichen Mengenverhältnissen eingesetzt werden sowie schleimbildende Stoffe und Verdickungsmittel, z. B. Hydroxyethyl- oder Hydroxypropylcellulose, Polyacrylsäure, Polyvinylpyrrolidon, daneben aber auch in kleinen Mengen cyclische Silikonöle (Polydimethylsiloxane) sowie flüssige Polymethylphenylsiloxane niedriger Viskosität.

Es ist ebenfalls vorteilhaft, kosmetischen oder dermatologischen Zubereitungen im Sinne der vorliegenden Erfindung übliche Antioxidantien zuzufügen. Erfindungsgemäß können als günstige Antioxidantien alle für kosmetische und/oder dermatologische Anwendungen geeigneten oder gebräuchlichen Antioxidantien verwendet werden.

Vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Carnosin, L-Carnosin und deren Derivate (z. B. Anserin), Carotinoide, Carotine (z. B. α-Carotin, β-Carotin, Lycopin) und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Zitronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Alanindiessigsäure, Flavonoide, Polyphenole, Catechine, Ubichinon und Ubichinol und deren Derivate, Vitamin C und Derivate (z. B. Ascorbylpalmitat, Mg-Ascorbylphosphat, Ascorbylacetat), Tocopherole und Derivate (z. B. Vitamin-E-acetat), sowie Koniferylbenzoat des Benzoëharzes, Rutinsäure und deren Derivate, Ferulasäure und deren Derivate, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, ZnSO₄) Seien und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Sofern die kosmetische oder dermatologische Zubereitung im Sinne der vorliegenden Erfindung eine Lösung oder Emulsion oder Dispersion darstellt, können als Lösungsmittel verwendet werden:
- Wasser oder wäßrige Lösungen
- Öle, wie Triglyceride der Caprin- oder der Caprylsäure, vorzugsweise aber Rizinusöl;
- Fette, Wachse und andere natürliche und synthetische Fettkörper, vorzugsweise Ester von Fettsäuren mit Alkoholen niedriger C-Zahl, z. B. mit Isopropanol, Propylenglykol oder Glycerin, oder Ester von Fettalkoholen mit Alkansäuren niedriger C-Zahl oder mit Fettsäuren;
- Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Insbesondere werden Gemische der vorstehend genannten Lösungsmittel verwendet. Bei alkoholischen Lösungsmitteln kann Wasser ein weiterer Bestandteil sein.

Die Ölphase der Emulsionen, Oleogele bzw. Hydrodispersionen oder Lipodispersionen im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkybenzoat und 2-Ethylhexylisostearat, Mischungen aus C₁₂₋₁₅-Alkybenzoat und Isotridecylisononanoat sowie Mischungen aus C₁₂₋₁₅-Alkybenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Emulsionen entsprechend der vorliegenden Erfindungen enthalten einen oder mehrere Emulgatoren, insbesondere vorteilhaft gewählt aus der Gruppe Glycerylstearat im Gemisch mit Ceteareth-20; Sorbitanstearat; Sorbitanoleat; Ceteareth-25; Ceteareth-6 im Gemisch mit Stearylalcohol; Cetylstearylalcohol im Gemisch mit PEG-40-Ricinusöl und Natriumcetylstearylsulfat; Triceteareth-4 Phosphat; Glycerylstearat; Natriumcetylstearylsulfat; Lecithin; Trilaureth-4 Phosphat; Laureth-4 Phosphat; Stearinsäure; Propylenglycolstearat SE; PEG-25-hydriertes Ricinusöl; PEG-54-hydriertes Ricinusöl; PEG-6 Caprylsäure/Caprinsäureglyceride; Sorbitanstearat; Glyceryloleat im Gemisch mit Propylenglycol; PEG-9-Stearat; Glyceryllanolat; Ceteth-2; Ceteth-20; Polysorbat 60; Lanolin; Glycerylstearat im Gemisch mit PEG-100 Stearat; Glycerylmyristat; mikrokristallines Wachs (Cera microcristallina) im Gemisch mit Paraffinöl (Paraffinum liquidum), Ozokerit, hydriertem Ricinusöl, Glyceryl Isostearat und Polyglyceryl-3-Oleat; Glyceryllaurat; PEG-40-Sorbitanperoleat; Laureth-4; Ceteareth-3; Wollwachssäuregemische; Isostearylglycerylether; Cetylstearylalkohol im Gemisch mit Natrium Cetylstearylsulfat; Wollwachsalkoholgemische; Laureth-23; Steareth-2; Glycerylstearat im Gemisch mit PEG-30 Stearat; PEG-40-Stearat; Glycol Distearat; PEG-22-Dodecyl Glycol Copolymer; Polyglyceryl-2-PEG-4 Stearat; Pentaerythrithylisostearat; Polyglyceryl-3 Diisostearat; Ceteareth-20; Sorbitan Oleat im Gemisch mit hydriertem Ricinusöl, Bienenwachs (Cera alba) und Stearinsäure; Natriumdihydroxycetylphosphat im Gemisch mit Isopropylhydroxycetylether; Methylglucosesesquistearat; Steareth-10; PEG-20-Stearat; Steareth-2 im Gemisch mit PEG-8 Distearat; Steareth-21; Steareth-20; Isosteareth-20; Methylglucosedioleat; PEG-7-hydriertes Ricinusöl; Sorbitanoleat im Gemisch mit PEG-2-hydriertem Ricinusöl, Ozokerit und hydriertem Ricinusöl; Sorbitanisostearat im Gemisch mit PEG-2-hydriertem Ricinusöl, Ozokerit und hydriertem Ricinusöl; PEG-45/ Dodecylglycol-Copolymer; Methoxy-PEG-22/Dodecylglycol-Copolymer; hydrierte Cocosfettsäureglyceride; Polyglyceryl-4-Isostearat; PEG-40-Sorbitanperoleat; PEG-40-Sorbitanperisostearat; PEG-20-Glycerylstearat; PEG-20-Glycerylstearat; PEG-8-Bienenwachs; Laurylmethiconcopolyol; Cetyldimethiconcopolyol; Polyglyceryl-2-laurat; Isostearyldiglycerylsuccinat; Stearamidopropyl-PG-dimoniumchloridphosphat; PEG-7-hydriertes Ricinusöl; Glycerylstearat, Ceteth-20; Triethylcitrat; PEG-20-Methylglucosesesquistearat; Ceteareth-12; Paraffinöl (Paraffinum liquidum); Glycerylstearatcitrat; Cetylphosphat; Sorbitansesquioleat; Acrylat/C₁₀₋₃₀-Alkylacrylat-Crosspolymer; Sorbitanisostearat; Methylglucosesesquistearat; Triceteareth-4-Phosphat; Trilaureth-4-Phosphat; Polyglycerylmethylglucosedistearat; Poloxamer 101; Kaliumcetylphosphat; Isosteareth-10; Polyglyceryl-2-sesquiisostearat; Ceteth-10; Polyglyceryl-2 Dipolyhydroxystearat; Oleth-20; Isoceteth-20; Glycerylisostearat; Polyglyceryl-3-Diisostearat; Glycerylstearat im Gemisch mit Ceteareth-20, Ceteareth-12, Cetylstearylalcohol und Cetylpalmitat; Cetylstearylalcohol im Gemisch mit PEG-20 Stearat; Glycerylstearat; PEG-30-Stearat.

Erfindungsgemäß verwendete Gele enthalten üblicherweise Alkohole niedriger C-Zahl, z. B. Ethanol, Isopropanol, 1,2-Propandiol, Glycerin und Wasser bzw. ein vorstehend genanntes Öl in Gegenwart eines Verdickungsmittels, das bei ölig-alkoholischen Gelen vorzugsweise Siliciumdioxid oder ein Aluminiumsilikat, bei wäßrig-alkoholischen oder alkoholischen Gelen vorzugsweise ein Polyacrylat ist.

Feste Stifte enthalten z. B. natürliche oder synthetische Wachse, Fettalkohole oder Fettsäureester.

Übliche Grundstoffe, welche für die Verwendung als kosmetische Stifte im Sinne der vorliegenden Erfindung geeignet sind, sind flüssige Öle (z. B. Paraffinöle, Ricinusöl, Isopropylmyristat), halbfeste Bestandteile (z. B. Vaseline, Lanolin), feste Bestandteile (z. B. Bienenwachs, Ceresin und mikrokristalline Wachse bzw. Ozokerit) sowie hochschmelzende Wachse (z. B. Carnaubawachs, Candelillawachs).

Kosmetische Zubereitungen im Sinne der vorliegenden Erfindung können auch als Gele vorliegen, die neben einem wirksamen Gehalt an der erfindungsgemäßen Wirkstoffkombination und dafür üblicherweise verwendeten Lösungsmitteln, bevorzugt Wasser, noch organische Verdickungsmittel, z. B. Gummiarabikum, Xanthangummi, Natriumalginat, Cellulose-Derivate, vorzugsweise Methylcellulose, Hydroxymethylcellulose, Hydroxyethylcellulose, Hydroxypropylcellulose, Hydroxypropylmethylcellulose oder anorganische Verdickungsmittel, z. B. Aluminiumsilikate wie beispielsweise Bentonite, oder ein Gemisch aus Polyethylenglykol und Polyethylenglykolstearat oder -distearat, enthalten. Das Verdickungsmittel ist in dem Gel z. B. in einer Menge zwischen 0,1 und 30 Gew.-%, bevorzugt zwischen 0,5 und 15 Gew.-%, enthalten.

Zubereitungen gemäß der vorliegenden Erfindung können sich auch durch einen Gehalt an Tensiden auszeichnen. Geeignet sind im Prinzip alle anionischen, kationischen, amphoteren und nichtionischen Tenside.

Vorteilhaft zu verwendende anionische Tenside sind
Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoylhydrolysiertes Soja Protein und Natrium-/ Kalium Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. Alaninate

Carbonsäuren und Derivate, wie
1. Carbonsäuren, beispielsweise Laurinsäure, Aluminiumstearat, Magnesiumalkanolat und Zinkundecylenat, und Derivate, wie z. B. Acyllactylate, Lauroyllactylat, Caproyllactylat,
2. Ester-Carbonsäuren, beispielsweise Calciumstearoyllactylat, Laureth-6 Citrat und Natrium PEG-4 Lauramidcarboxylat,
3. Ether-Carbonsäuren, beispielsweise Natriumlaureth-13 Carboxylat und Natrium PEG-6 Cocamide Carboxylat,

Phosphorsäureester und Salze, wie beispielsweise DEA-Oleth-10-Phosphat und Dilaureth-4 Phosphat,

Sulfonsäuren und Salze, wie
1. Acyl-isethionate, z. B. Natrium-/ Ammoniumcocoyl-isethionat,
2. Alkylarylsulfonate,
3. Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat
sowie
Schwefelsäureester, wie
1. Alkylethersulfat, beispielsweise Natrium-, Ammonium-, Magnesium-, MIPA-, TIPA-Laurethsulfat, Natriummyrethsulfat und Natrium C₁₂₋₁₃ Parethsulfat,
2. Alkylsulfate, beispielsweise Natrium-, Ammonium- und TEA- Laurylsulfat.

Vorteilhaft zu verwendende kationische Tenside sind
1. Alkylamine,
2. Alkylimidazole,
3. Ethoxylierte Amine und
4. Quaternäre Tenside.
5. Esterquats

Zubereitungen gemäß der vorliegenden Erfindung können ferner vorteilhaft weitere quaternäre Ammoniumverbindungen enthalten, insbesondere solche, welche als quaternäre Tenside bezeichnet werden. Vorteilhaft zu verwenden sind Alkylbetain, Alkylamidopropylbetain und Alkylamidopropylhydroxysulfain, Alkylamidethyltrimethylammoniumethersulfate, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes/ propoxyliertes Lanolin, ethoxylierte/ propoxylierte Polysiloxane, propoxylierte POE-Ether und Alkylpolyglycoside wie Laurylglucosid, Decylglycosid und Cocoglycosid.
6. Sucroseester, -Ether
7 Polyglycerinester, Diglycerinester, Monoglycerinester
8. Methylglucosester, Ester von Hydroxysäuren

Vorteilhaft ist ferner die Verwendung einer Kombination von anionischen und/oder amphoteren Tensiden mit einem oder mehreren nicht-ionischen Tensiden.

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

Der Nachweis der Wirksamkeit wurde an menschlichen Probanden mit Hilfe der sogenannten "Imprintmethode" geführt. Bei dieser Methode wird das zu untersuchende Material am Unterarm innen mehrere Tage hintereinander (topisch) angewendet, sodann begeben sich die Probanden in die Sauna zum Schwitzen. An der Untersuchungsstelle wird eine Silikonmasse aufgetragen, die während der Schwitzphase aushärtet. An den Stellen, an denen Schweisströpfchen austreten, bilden sich Abdrücke in der Silikonmasse, welche bildanalytisch ausgewertet werden können. Nach diesem Verfahren wurden zunächst die Wirkstoffkombinationen in wäßriger Lösung untersucht und sodann entsprechende kosmetische Zubereitungen.

Die desodorierende Wirkung wurde an menschlichen Probanden durch den sogenannten "Sniff"-Test an den Achselhöhlen ermittelt. Die bakterizide und fungizide Wirksamkeit wurde durch in vitro Tests, Suspensionstests und Hemmhoftests und an einigen Beispielen auch an menschlichen Probanden durch Abstriche vor und nach Applikation ermittelt.

### Beispiele:

### Beispiel 1 Fußspray

Eine Lösung wurde hergestellt aus

| | |
|---|---|
| 90,75 Gew.-% | 2-Propanol |
| 6,00 Gew.-% | Aluminiumchlorhydrat |
| 1,00 Gew.-% | Hexadecyldimethylethylammoniumethylsulfat |
| 1,00 Gew.-% | Glycerol, 86,5 %ig |
| 1,00 Gew.-% | Bentonite |
| 0,10 Gew.-% | Farnesol |
| 0,10 Gew.-% | Menthol |
| 0,05 Gew.-% | Parfüm |

### Beispiel 2 Fuß-Gel

Eine viskose Lösung wurde hergestellt aus

| | |
|---|---|
| 86,30 Gew.-% | Wasser |
| 10,00 Gew.-% | Aluminiumchlorhydrat |
| 1,00 Gew.-% | Hydroxyethylcellulose |
| 0,80 Gew.-% | Dexpanthenol |
| 0,04 Gew.-% | Wollwachsalkohol |
| 0,07 Gew.-% | Tetradecanol-1 |
| 0,14 Gew.-% | 2-Ethylhexyl-2-ethylhexanoat |
| 0,15 Gew.-% | Heptamethylnonan |
| 0,20 Gew.-% | Minzöl |
| 0,30 Gew.-% | Menthol |
| 1,00 Gew.-% | Hexadecyldimethylethylammoniumethylsulfat |

### Beispiel 3 Fußcreme

Eine Creme wurde hergestellt aus

| | |
|---|---|
| 20,00 Gew.-% | Aluminiumchlorhydrat |
| 1,00 Gew.-% | Hexadecyldimethylethylammoniumethylsulfat |
| 55,39 Gew.-% | Wasser |
| 0,75 Gew.-% | Dexpanthenol |
| 0,30 Gew.-% | Minzöl |
| 3,50 Gew.-% | Decamethylcyclopentasiloxan |
| 0,20 Gew.-% | nichtionisches, hydrophiles Bienenwachs, (mit PEG verestert) |
| 3,50 Gew.-% | 1-Hexadecanol |
| 3,00 Gew.-% | Polyoxyethylen-(40)-Stearat |
| 3,50 Gew.-% | Di-n-Octylether |
| 3,50 Gew.-% | Capryl-/Caprinsäureester von ungesättigten Fettsäuren |
| 2,50 Gew.-% | Gemisch aus Mono-, Di-, und Triglyceriden der Stearin und Palmitinsäure |
| 2,00 Gew.-% | Triethylcitrat |
| 0,30 Gew.-% | Anisalkohol |
| 0,06 Gew.-% | Anissäure |
| 0,50 Gew.-% | Menthol |

## Patentansprüche

1. Synergistische Wirkstoffkombinationen aus
a) mindestens einer quaternären Ammoniumverbindung gewählt aus der Gruppe, welche gebildet wird aus quaternären Ammoniumverbindungen der allgemeinen Strukturformel 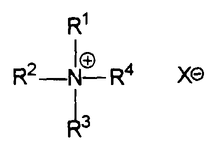 und Alkylpyridiniumsalzen der allgemeinen Strukturformel 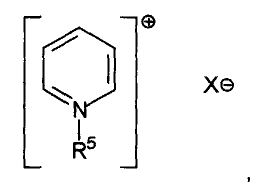 und
b) mindestens einem anorganischen Antitranspirantwirkstoff
wobei R¹ und R² unabhängig voneinander Methyl- oder Ethylgruppen darstellen, R³ gewählt wird aus der Gruppe der Alkylreste mit 1 bis 18 Kohlenstoffatomen, R⁴ gewählt wird aus der Gruppe, welche gebildet wird aus den Alkylresten mit 8 bis 18 Kohlenstoffatomen und den Aralkylresten mit 7 bis 18 Kohlenstoffatomen, R⁵ wird gewählt aus der Gruppe der Alkylreste mit 10 bis 18 Kohlenstoffatomen und worin X⁻ein anorganisches oder organisches Anion darstellt.

2. Kombination nach Anspruch 1, dadurch gekennzeichnet, daß die quaternäre(n) Ammoniumverbindung(en) aus der Gruppe Decyldimethylethylammoniumethylsulfat, Dodecyldimethylethylammoniumethylsulfat und Hexadecyldimethylethylammoniumethylsulfat gewählt wird/werden.

3. Kombination nach Anspruch 1, dadurch gekennzeichnet, daß der oder die anorganische(n) Antitranspirantwirkstoff(e) aus der Gruppe der Aluminiumsalze, Zirkoniumsalze und Zinksalze gewählt wird/werden.

4. Kombination nach Anspruch 1, dadurch gekennzeichnet, daß der oder die anorganische(n) Antitranspirantwirkstoff(e) aus der Gruppe Aluminiumchlorid, Aluminiumhydroxychlorid, Aluminium-Zirkoniumchlorid, Aluminium-Zirkonium-Chlorhydrat, Aluminium-Zirkoniumsulfat, Aluminiumnitrat, Aluminiumlactat, Aluminiumallantoinat, Aluminiumacetat, Aluminiumsulfat, Zinkchlorid, Zinksulfat, Zinknitrat, Zinkacetat, Zinklactat und Zinkallantoinat gewählt wird/werden.

5. Kombination nach Anspruch 1, dadurch gekennzeichnet, daß das Verhältnis von quaternärer Ammoniumverbindung (eine oder mehrere Verbindungen) zu anorganischem Antitranspirantwirkstoff (eine oder mehrere Verbindungen) in der Wirkstoffkombination aus dem Bereich 3 : 997 bis 30 : 70 gewählt wird.

6. Kosmetische oder dermatologische Fußpflegezubereitung, dadurch gekennzeichnet daß sie eine Wirkstoffkombination aus
a) mindestens einer quaternären Ammoniumverbindung gewählt aus der Gruppe, welche gebildet wird aus quaternären Ammoniumverbindungen der allgemeinen Strukturformel 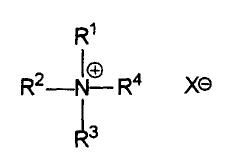 und Alkylpyridiniumsalzen der allgemeinen Strukturformel 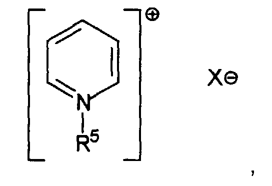 und
b) mindestens einem anorganischen Antitranspirantwirkstoff enthält,
wobei R¹ und R² unabhängig voneinander Methyl- oder Ethylgruppen darstellen, R³ gewählt wird aus der Gruppe der Alkylreste mit 1 bis 18 Kohlenstoffatomen, R⁴ gewählt wird aus der Gruppe, welche gebildet wird aus den Alkylresten mit 8 bis 18 Kohlenstoffatomen und den Aralkylresten mit 7 bis 18 Kohlenstoffatomen, R⁵ wird gewählt aus der Gruppe der Alkylreste mit 10 bis 18 Kohlenstoffatomen und worin X⁻ein anorganisches oder organisches Anion darstellt.

7. Zubereitung nach Anspruch 6, dadurch gekennzeichnet, daß der Gehalt an der oder den quaternären Ammoniumverbindung(en) aus dem Bereich zwischen 0,01 und 2,0 Gew.-% und der Gehalt an dem oder den anorganischen Antitranspirantwirkstoff(en) aus dem Bereich zwischen 5,0 und 30 Gew.-% gewählt wird, jeweils bezogen auf das Gesamtgewicht der Zusammensetzung.

8. Zubereitung nach Anspruch 6, dadurch gekennzeichnet, daß zusätzlich Tenside und/oder Hilfs-, Zusatz- und/oder weitere Wirkstoffe enthalten sind.

9. Verwendung von Wirkstoffkombinationen nach Anspruch 1 als Konservierungsmittel, insbesondere als Konservierungsmittel in kosmetischen oder dermatologischen Zubereitungen.
